# EUROPEAN PATENT SPECIFICATION

(11) **EP 2 576 076 B1**
(45) Date of publication and mention of the grant of the patent: **19.12.2018**
(21) Application number: 11718199.0
(22) Date of filing: 22.04.2011
(51) Int. Cl.: B04B 5/04, B04B 9/14, A61M 1/02, A61M 1/36, A61J 1/10

(54) **APPARATUS AND METHOD FOR OPERATING A MULTI-UNIT BLOOD PROCESSOR WITH VARYING UNITS OF BLOOD**
VORRICHTUNG UND VERFAHREN ZUM BETRIEB EINES BLUTPROZESSORS MIT MEHREREN EINHEITEN MIT UNTERSCHIEDLICHEN BLUTEINHEITEN
APPAREIL ET PROCÉDÉ DE FONCTIONNEMENT D'UN DISPOSITIF DE TRAITEMENT DE SANG À MULTIPLES UNITÉS DOTÉ DE DIVERSES UNITÉS DE SANG

(30) Priority: 26.05.2010 US 348525 P
(43) Date of publication of application: 10.04.2013
(73) Proprietor: Terumo BCT, Inc., Lakewood, CO 80215 (US)
(72) Inventor: DOLECEK, Victor D., Englewood Colorado 80111 (US); HUDOCK, Darryl, Highlands Ranch Colorado 80129 (US); MESSENGER, Paul, Littleton Colorado 80123 (US)
(74) Representative: J A Kemp
(86) International application number: PCT/US2011/033537
(87) International publication number: WO 2011/149611

(56) References cited:
- JP-A- 2007 283 093
- US-A1- 2005 048 033
- US-A1- 2007 284 320
- US-B2- 7 674 221

## Description

The present invention relates to an apparatus and a method for separating at least two discrete volumes of a blood into at least two components each in a multi-unit blood processor.

US Patent 7,674,221 describes an apparatus for separating discrete volumes of a composite liquid such as blood into at least two components.

The apparatus and a method of the invention are particularly appropriate for the separation of biological fluids comprising an aqueous component and one or more cellular components, such as red blood cells, white blood cells or platelets. Optionally, a component, such as red blood cells, may be washed to remove certain biologic components such as prions. A component, such as washed red blood cells, may also be filtered so as to remove residual prions, white blood cells or platelets from the red blood cells.

An apparatus for processing blood components that can process at once at least two discrete volumes of a composite liquid, in particular, two discrete volumes that may be not the same, and wherein the proportions of the various components of the composite liquid that may vary from one discrete volume to another one, is known from US 2008-0090714. A method is described therein for separating at least two discrete volumes of a composite liquid into at least a first component and a second component comprising enclosing in at least two separation cells mounted on a rotor at least two separation bags containing two discrete volumes of a composite liquid respectively; storing in at least one container included in the rotor at least two first component bags connected to the at least two separation bags respectively; rotating the rotor at a sedimentation speed at which the at least a first and a second components sediment in each of the separation bags; transferring at least one fraction of a first separated component from the at least two separation bags into the at least two first component bags connected thereto respectively; detecting a characteristic of a component at a first determined location in each separation bag; and stopping transferring the at least one fraction of the first component from each separation bag into the first component bag connected thereto, upon detection of the characteristic of a component at the first determined location.

A multi-unit processor as described in U.S. Patent 7,674,221 uses a hydraulic bladder system to express blood components out of a whole blood bag and into various collection bags. The hydraulic bladder system relies on the presence of whole blood bags in each of the processing areas to prevent the bladder system from becoming unbalanced by forcing too much hydraulic fluid into an empty processing area. A blood bank or other blood processing organization will not always have sufficient units of blood for processing in each of the processing areas of the multi-unit blood processor. The invention is defined in the claims.

According to the present invention, a dummy whole blood bag, containing an inert fluid such as water or saline solution, is placed in one or more of the processing locations. The dummy bag has characteristics that allow the blood processing apparatus to recognize the presence of the dummy bag. The blood processing apparatus then performs separation procedures only at those processing areas containing blood components, and not on the areas containing a dummy bag.

Several methods for dummy bag recognition may be used. In the apparatus described herein, photo sensors are used to control the separation process at each separation area. In one embodiment, an opaque dummy bag may be detected by a photo sensor. In another embodiment, the dummy bag may have a short stub of tubing adapted to be placed next to a tubing photo sensor, which is otherwise used to detect the presence of various blood components in a tube. The stub of tubing may be opaque or have optical characteristics that are unique as compared to blood or a blood component. In yet another embodiment, a specialized sensor, such as a radio frequency sensor, on the blood processing apparatus, may be used to detect a passive radio frequency emitter on the dummy bag. In a further embodiment, the dummy bag may have a bar code label that could be read by a bar code reader used to track units of blood through a separation process. Once the blood processing apparatus has determined that a dummy bag has been placed in one or more blood processing areas, the apparatus will not attempt to process the areas with a dummy bag.

In connection herewith, a blood processing apparatus is provided that is capable of separating at least two discrete volumes of a composite liquid into at least a first component and a second component and optionally washing at least one component, the apparatus comprising a centrifuge having a rotor with a rotation axis, at least two separation cells mounted on the rotor, each cell adapted to receive a separation bag containing a volume of composite liquid, such as blood; and at least one sensor associated with each separation cell for generating information related to a characteristic of a component separated in a separation bag within the separation cell; and a control unit programmed for receiving information generated by the at least one sensor associated with each separation cell; and for controlling rotation speed in view of information generated by one of the at least one sensor associated with each of the at least two separation cells. The apparatus is adapted to receive a disposable set of tube-connected bags. The disposable set preferably comprises a primary bag, initially containing whole blood, fluidly connected to at least one (preferably two) component bag for receiving blood components such as plasma or platelets and optionally fluidly connected to a wash solution bag and a wash solution discard bag. The disposable set may further comprise a red blood cell collection bag fluidly connected to the primary bag through a filter. A dummy bag is provided to replace a disposable set of tube-connected, blood containing bags, if all separation cells cannot be filled with such disposable sets.

The apparatus comprises a plurality of valves associated with each separation cell. The valves comprise at least one valve adapted to control fluid flow into the at least one component bag, more preferably two valves where two component bags are provided, each component valve being associated with a component bag. The valves may optionally further comprise a wash solution valve for controlling fluid flow out of the wash solution bag and a discard valve for controlling fluid flow of used wash solution into the wash solution discard bag.

Other features and advantages of the invention will appear from the following description and accompanying drawings, which are to be considered exemplary only.
Figure 1 is a schematic view of a first set of bags designed for cooperating with a separation apparatus.
Figure 2 is a schematic view, partly in cross-section along a diametric plane, of a first embodiment of a separation apparatus.
Figure 3 is a top view of the rotor of the separation apparatus of Figure 2.
Figure 4 is schematic view, in cross-section along a radial plane, of a separation cell of the separation apparatus of Figures 2 and 3.
Figure 5 is a perspective view of a rotor of a second embodiment of a separation apparatus.
Figure 6 is a cross-section view of the rotor of Figure 5, along a diametric plane.
Figure 7 is a top view of the rotor of Figure 5.
Figure 8 is a plan view of a dummy bag for use in the separation apparatus.
Figure 9 is a schematic diagram of a control program.

For the sake of clarity, the invention will be described with respect to a specific use, namely the separation of whole blood into at least two components, in particular into a plasma component and a red blood cell component, or into a plasma component, a platelet component and a red blood cell component. The discrete volume mentioned hereunder will typically be the volume of a blood donation. The volume of a blood donation may vary from one donor to another one (450ml plus or minus 10%). It is also recalled that the proportion of the components of blood usually varies from one donor to another one, in particular the hematocrit, which is the ratio of the volume of the red blood cells to the volume of the sample of whole blood considered. In other words the density of blood may slightly vary for one donor to another one. It should be understood however that this specific use is exemplary only.

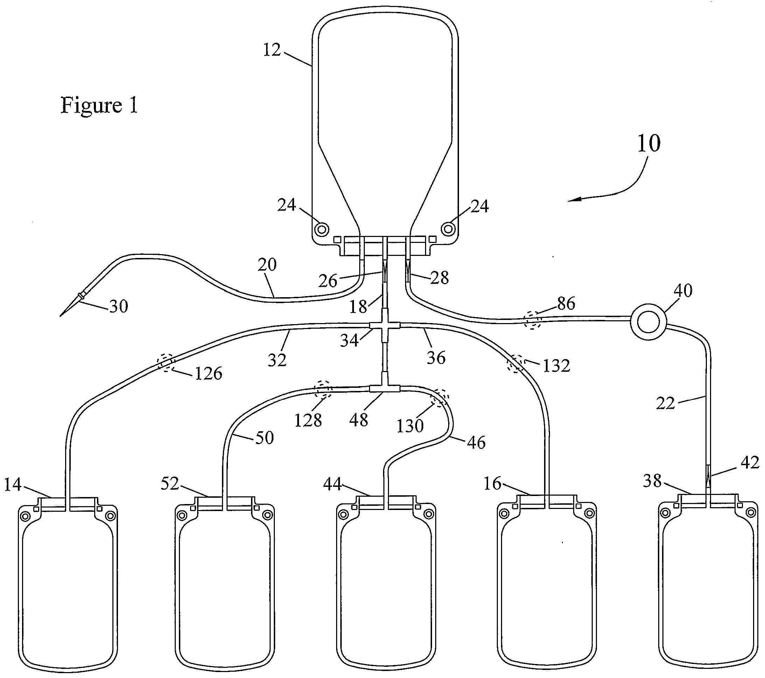

Figure 1 shows an example of a set 10 of bags adapted to be used for the separation of a composite liquid (e.g. whole blood) into at least one component (e.g. plasma, platelets, or both) and a second component (e.g. red blood cells). This bag set comprises a flexible primary separation bag 12 and two flexible component bags 14, 16 connected thereto.

When the composite liquid is whole blood, the separation bag 12 has two purposes, and is successively used as a collection bag and as a separation bag. It is intended to initially receive a discrete volume of whole blood from a donor (usually about 450 ml) and to be used later as a separation chamber in a separation apparatus. The separation bag 12 is flat and generally rectangular. It is made of two sheets of plastic material that are welded together so as to define there between an interior space having a main rectangular portion connected to a triangular top portion. A first tube 18 is connected to the top of the triangular portion, and a second tube 20 and a third tube 22 are connected on opposite sides adjacent the first tube 18. The proximal ends of the three tubes 18, 20, 22 are embedded between the two sheets of plastic material so as to be parallel. The separation bag 12 further comprises a hole 24 in each of its two proximal corners that are adjacent to the three tubes 18, 20, 22. The holes 24 are used to secure the separation bag to a separation cell, as will be described later.

The separation bag initially contains a volume of anti-coagulant solution (typically about 63 ml of a solution of citrate phosphate dextrose for a blood donation of about 450 ml). The first and third tubes 18, 22 are fitted at their proximal ends with a breakable stopper 26, 28 respectively, blocking liquid flow therethrough. The second tube 20 is a collection tube having a needle 30 connected to its distal end. At the beginning of a blood donation, the needle 30 is inserted in the vein of a donor and blood flows into the separation bag 12. After a desired volume of blood has been collected in the separation bag 12, the collection tube 20 is sealed and cut, disconnecting the needle from the bag set 10. Alternatively, previously collected blood may be transferred to the separation bag 12 through the collection tube 20, with or without the use of the needle 30.

The first component bag 14 is intended for receiving a plasma component. It is flat and substantially rectangular. It is connected through a plasma collection tube 32 and an X-connector 34 to the first tube 18. The second component bag 16 is intended for receiving a platelet component. It is also flat and substantially rectangular. It is connected through a platelet collection tube 36 and the X-connector 34 to the first tube 18. A third component bag 38 is intended to receive an optional washed red blood cell component, if washing is desired, from the primary bag 12. Washed red blood cells may be drained through tube 22, which may include a filter 40, into third component bag 38. A breakable stopper 42 in tube 22 prevents premature flow of red blood cells into the third component bag 38.

An optional wash solution bag 44 initially contains wash solution such as saline or a storage solution such as FAGM. Wash solution may be transferred through a wash solution tube 46 and a T-connector 48 by way of the first tube 18 into the primary bag 12 when the primary bag 12 contains high hematocrit blood cells. "High hematocrit" means a percentage of red blood cell volume to total fluid volume of at least 80 percent, more preferably 90 percent, and yet more preferably 95 percent. After wash solution is mixed with high hematocrit red blood cells and subsequently separated, used wash solution may be extracted through the first tube 18, T-connector 48, and discard tube 50 into a wash solution discard bag 52.

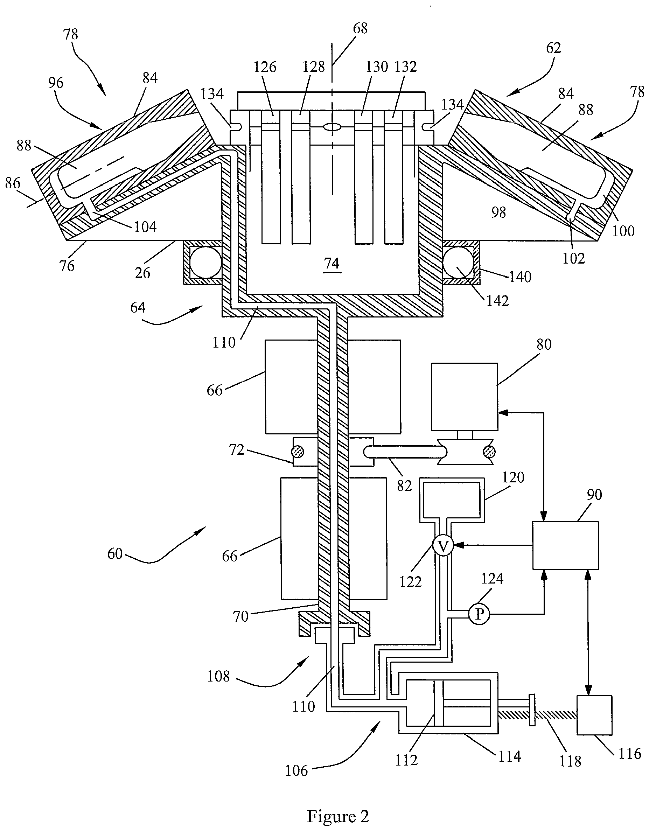

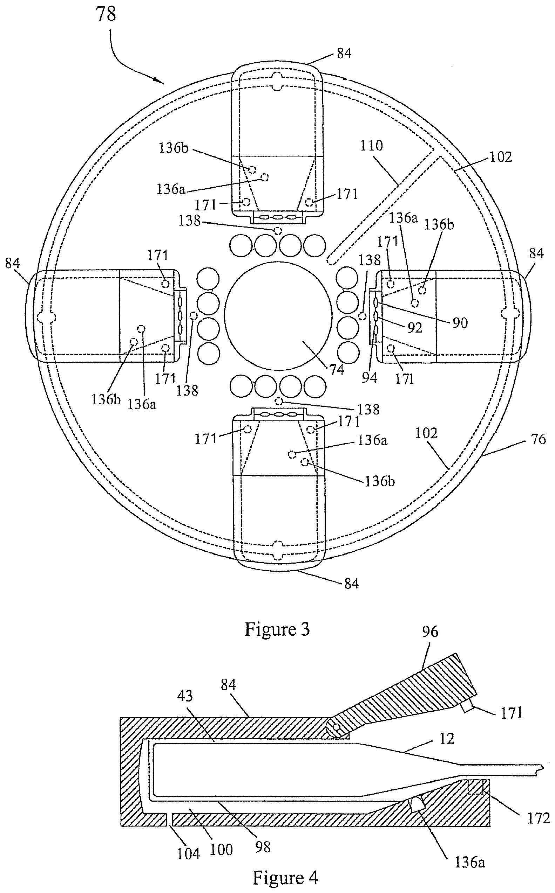

Figures 2 and 3 show a first embodiment of an apparatus 60 for simultaneously separating by centrifugation four discrete volumes of a composite liquid. The apparatus comprises a centrifuge 62 adapted to receive four of the sets 10 of bags shown in Figure 1, with the four discrete volumes of a composite liquid contained in the four primary separation bags 12; a component transferring means for transferring at least one separated component from each separation bag into a component bag connected thereto; and optional means for washing a residual high hematocrit red blood cell component.

The centrifuge 62 comprises a rotor 64 that is supported by a bearing assembly 66 allowing the rotor 64 to rotate around a rotation axis 68. The rotor comprises a cylindrical rotor shaft 70 to which a pulley 72 is connected; a storage means comprising a central cylindrical container 74 for containing component bags, which is connected to the rotor shaft 70 at the upper end thereof so that the longitudinal axis of the rotor shaft 70 and the longitudinal axis of the container 74 coincide with the rotation axis 68, and a frusto-conical turntable 76 connected to the upper part of the central container 74 so that its central axis coincides with the rotation axis 68. The frusto-conical turntable 76 flares underneath the opening of the container 74. Four identical separation cells 78 are mounted on the turntable 76 so as to form a symmetrical arrangement with respect to the rotation axis 68. The centrifuge further comprises a motor 80 coupled to the rotor by a belt 82 engaged in a groove of the pulley 72 so as to rotate the rotor about the rotation axis 68.

Each separation cell 78 comprises a container 84 having the general shape of a rectangular parallelepiped. The separation cells 78 are mounted on the turntable 76 so that their respective median longitudinal axes 86 intersect the rotation axis 68, so that they are located substantially at the same distance from the rotation axis 68, and so that the angles between their median longitudinal axes 86 are substantially the same (e.g. 90 degrees). The exact position of the separation cells 78 on the turntable 76 is adjusted so that the weight on the turntable is equally distributed when the separation cells 78 are empty, that is, so that the rotor is balanced. It results from the arrangement of the separation cells 78 on the turntable 76 that the median axis 86 of the separation cells 78 are inclined downwardly with respect to a plane perpendicular to the rotation axis 68.

Each container 84 comprises a cavity 88 that is so shaped and dimensioned as to loosely accommodate a separation bag 12 full of liquid, of the type shown in Figure 1. The cavity 88 (which will be referred to later also as the "separation compartment") is defined by a bottom wall, which is the farthest to the rotation axis 68, a lower wall that is the closest to the turntable 76, an upper wall opposite to the lower wall, and two lateral walls. The cavity 88 comprises a main part, extending from the bottom wall, which has substantially the shape of a rectangular parallelepiped with rounded corners and edges, and an upper part, which has substantially the shape of a prism having convergent triangular bases. In other words, the upper part of the cavity 88 is defined by two sets of two opposing walls converging towards the central median axis 86 of the container 84. One interesting feature of this design is that it causes a radial dilatation of a thin layer of a minor component of a composite fluid (e.g. the platelets in whole blood) after separation by centrifugation, and makes the layer more easily detectable in the upper part of a separation bag. The two couples of opposite walls of the upper part of the separation cell 78 converge towards three cylindrical parallel channels 90, 92, 94 (see Fig. 3), opening at the top of the container 84, and through which, when a separation bag 12 is set in the container 84, the three tubes 18, 20, 22 extend.

The container 84 also comprises a hinged lateral lid 96, which is comprised of an upper portion of the external wall of the container 84, i.e. the wall that is opposite to the turntable 76. The lid 96 is so dimensioned as to allow, when open, an easy loading of a separation bag 12 full of liquid into the separation cell 78. The container 84 comprises a locking means (not shown) by which the lid 96 can be locked to the remaining part of the container 84. The container 84 also comprises a securing means for securing a separation bag 12 within the separation cell 78. The bag securing means comprises two pins 171 (see Fig. 3 and Fig. 4) protruding on the internal surface of the lid 96, close to the top of separation cell 78, and two corresponding recesses 172 in the upper part of the container 84. The two pins are so spaced apart and dimensioned as to fit into the two holes 24 in the upper corners of a separation bag 12.

The separation apparatus further comprises a component transferring means for transferring at least one separated component from each separation bag into a component bag connected thereto. The component transferring means comprises a squeezing system for squeezing the separation bags 12 within the separation compartments 88 and causing the transfer of separated components into component bags 14,16. The squeezing system comprises a flexible diaphragm 98 that is secured to each container 84 so as to define an expandable chamber 100 in the cavity thereof. More specifically, the diaphragm 98 is dimensioned so as to line the bottom wall of the cavity 88 and a large portion of the lower wall of the cavity 88, which is the closest to the turntable 76. The squeezing system further comprises a peripheral circular manifold 102 that forms a ring within the turntable 76 extending close to the periphery of the turntable 76. Each expansion chamber 100 is connected to the manifold 102 by a supply channel 104 that extends through the wall of the respective container 84, close to the bottom thereof. The squeezing system further comprises a hydraulic pumping station 106 for pumping a hydraulic liquid in and out the expandable chambers 100 within the separation cells 78. The hydraulic liquid is selected so as to have a density slightly higher than the density of the more dense of the components in the composite liquid to be separated (e.g. the red blood cells, when the composite liquid is blood). As a result, during centrifugation, the hydraulic liquid within the expandable chambers 100, whatever the volume thereof, will generally remain in the most external part of the separation cells 78. The pumping station 106 is connected to the expandable chambers 100, through a rotary seal 108, by a duct 110 that extends through the rotor shaft 70, the bottom and lateral wall of the central container 74, and, from the rim of the central container 74, radially through the turntable 76 where it connects to the manifold 102. The pumping station 106 comprises a piston pump having a piston 112 movable in a hydraulic cylinder 114 fluidly connected via the rotary seal or fluid coupling 108 to the rotor duct 110. The piston 112 is actuated by a stepper motor 116 that moves a lead screw 118 linked to the piston rod. The hydraulic cylinder 114 is also connected to a hydraulic liquid reservoir 120 having an access controlled by a valve 122 for selectively allowing the introduction or the withdrawal of hydraulic liquid into and from a hydraulic circuit including the hydraulic cylinder 114, the rotor duct 110 and the expandable hydraulic chambers 100. A pressure gauge 124 is connected to the hydraulic circuit for measuring the hydraulic pressure therein. The stepper motor 116, motor 80, and valve 122 are controlled by a microprocessor controller 90. The controller 90 receives data from pressure sensor 124, as well as additional sensors 136a, 136b, and 138, and controls valves 122 and additional valves 126, 128, 130, and 132, as described below.

The separation apparatus further comprises four sets of four pinch valves 126, 128, 130, 132 that are mounted on the rotor around the opening of the central container 74. Each set of pinch valves 126, 128, 130, 132 faces one separation cell 78, with which it is associated. The pinch valves 126, 128, 130, 132 are designed for selectively blocking or allowing a flow of liquid through a flexible plastic tube, and selectively sealing and cutting a plastic tube. Each pinch valve 126, 128, 130, 132 comprises an elongated cylindrical body and a head having a groove 134 that is defined by a stationary upper jaw and a lower jaw movable between an open and a closed position. The groove 134 is so dimensioned that one of the tubes 32, 36, 46, 50 of the bag sets shown in Figure 1 can be snuggly engaged therein when the lower jaw is in the open position. The elongated body contains a mechanism for moving the lower jaw and it is connected to a radio frequency generator that supplies the energy necessary for sealing and cutting a plastic tube. The pinch valves 126, 128, 130, 132 are mounted inside the central container 74, adjacent the interior surface thereof, so that their longitudinal axes are parallel to the rotation axis 68 and their heads protrude above the rim of the container 74. The position of a set of pinch valves 126, 128, 130, 132 with respect to a separation bag 12 and the tubes 32, 36, 46, 50 connected thereto when the separation bag 12 rests in the separation cell 78 associated with this set of pinch valves 126, 128, 130, 132 is shown in doted lines in Figure 1. Electric power is supplied to the pinch valves 126, 128, 130, 132 through a slip ring array that is mounted around a lower portion of the rotor shaft 70. The pinch valves respond to commands from the controller 90 or a similar microprocessor mounted on the rotor 78.

The separation apparatus further comprises four sets of sensors 136a, 136b, and 138, (see Fig. 3), also connected to the controller 90 or to a similar microprocessor mounted on the rotor 78, for monitoring the separation of the various components occurring within each separation bag when the apparatus operates. A pair of sensors 136a, 136b is embedded in a curved portion of the container 84 opposite the lid 96 of the container 84 of each separation cell 78 such that light from an emitter 136a may be received at a receiver 136b through a portion of the separation bag 12. When a separation bag 12 rests in the container 84 and the lid 96 is closed, the first sensor or bag sensor 136a, 136b faces the upper triangular part of the separation bag 12. The bag sensor 136a, 136b is able to detect blood cells in a liquid. A tube sensor 138 is able to detect the presence of absence of liquid in the tube 18 as well as to detect blood cells in a liquid. Each sensor 136, 138 may comprise a photocell including an infrared LED and a photo-detector. Electric power is supplied to the sensors 136, 138 through the slip ring array that is mounted around the lower portion of the rotor shaft 70.

The separation apparatus further comprises a first balancing means for initially balancing the rotor when the weights of the four separation bags 12 contained in the separation cells 78 are different. The first balancing means substantially comprises the same structural elements as the elements of the component transferring means described above, namely: four expandable hydraulic chambers 100 interconnected by a peripheral circular manifold 102, and a hydraulic liquid pumping station 106 for pumping hydraulic liquid into the hydraulic chambers 100 through a rotor duct 110, which is connected to the circular manifold 102. In order to initially balance the rotor, whose four separation cells 40 contain four discrete volumes of a composite liquid that may not have the same weight (because the four volumes may be not equal, and/or the density of the liquid may slightly differ from one volume to the other one), the pumping station 106 is controlled so as to pump into the interconnected hydraulic chambers 100, at the onset of a separation process, a predetermined volume of hydraulic liquid that is so selected as to balance the rotor in the most unbalanced situation. For whole blood, the determination of this balancing volume takes into account the maximum difference in volume between two blood donations, and the maximum difference in hematocrit (i.e. in density) between two blood donations. Under centrifugation forces, the hydraulic liquid will distribute unevenly in the four separation cells 78 depending on the difference in weight of the separation bags 12, and balance the rotor. In order to get an optimal initial balancing, the volume of the cavity 88 of the separation cells 78 should be selected so that the cavities 88, whatever the volume of the separation bags 12 contained therein, are not full after the determined amount of hydraulic liquid has been pumped into the interconnected expansion chambers 100.

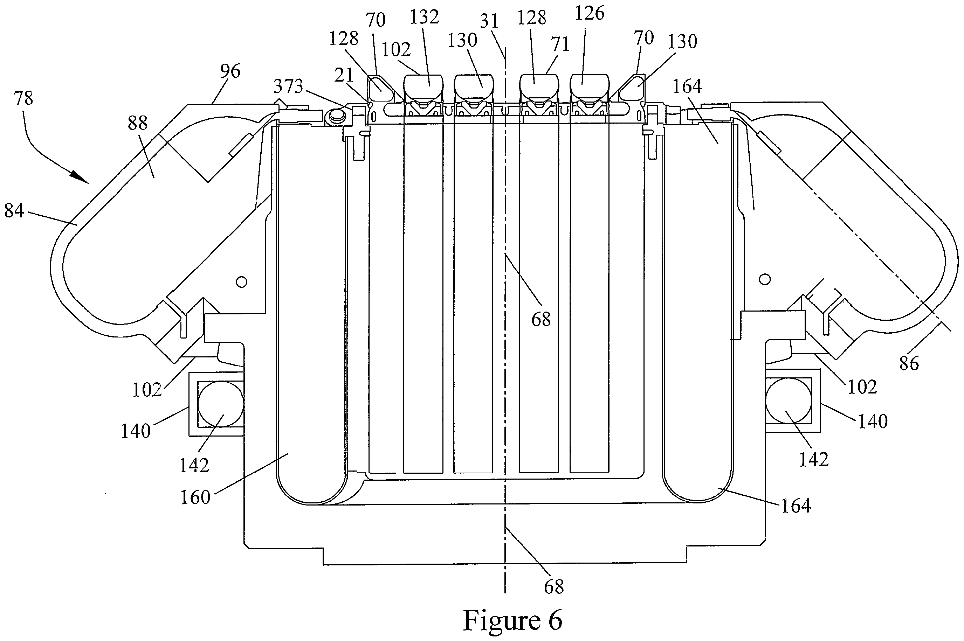

The separation apparatus further comprises a second balancing means, for balancing the rotor when the weights of the components transferred into the component bags 14, 16 in the central container 74 are different. For example, when two blood donations have the same hematocrit and different volumes, the volumes of plasma extracted from each donation are different, and the same is true when two blood donations have the same volume and different hematocrit. As shown in Figures 2, 5, and 6 the second balancing means comprises a balance assembly or ring 140, more particularly described in US Patent publication No. 2007-0284320. The balancing apparatus of the separation apparatus comprises one or two balancing assemblies, each including a series of ponderous satellites or balls that can move freely on a specific circular orbit centered on and perpendicular to the axis of rotation of the rotor. The weight of the ponderous satellite, the number of the satellites, and the diameter of the orbit on which the satellites are free to revolve are selected in view of 1) an anticipated maximum unbalance to be neutralized, 2) the distance from the axis of the rotor where the cause of the unbalance is to occur and 3) the space that is available on the rotor for mounting the balancing assembly. The balance assembly 140 comprises a ring-shaped housing defining a cavity whose cross-section, along a radial plane, is generally rectangular. The housing comprises a container for spherical ponderous satellites (balls) 123, which are housed in a cylindrical outer race, in which the balls slightly engage, and on which they roll, when the rotor rotates. The balancing assembly 140 comprises a plurality of balls. When the balls are in contact with each other, they occupy a sector of the ring of about 180 degrees. The balancing assembly 140 also comprises a damper or dampening fluid or element for providing resistance to the movement of the balls.

The separation apparatus further comprises a controller 90 including a control unit (e.g. a microprocessor) and a memory unit for providing the microprocessor with information and programmed instructions relative to various separation protocols (e.g. a protocol for the separation of a plasma component and a blood cell component, or a protocol for the separation of a plasma component, a platelet component, and a red blood cell component) and to the operation of the apparatus in accordance with such separation protocols. In particular, the microprocessor or controller 90 is programmed for receiving information relative to the centrifugation speed(s) at which the rotor is to be rotated during the various stages of a separation process (e.g. stage of component separation, stage of a plasma component expression, stage of suspension of platelets in a plasma fraction, stage of a platelet component expression, etc), and information relative to the various transfer flow rates at which separated components are to be transferred from the separation bag 12 into the component bags 14, 16. The information relative to the various transfer flow rates can be expressed, for example, as hydraulic liquid flow rates in the hydraulic circuit, or as rotation speeds of the stepper motor 116 of the hydraulic pumping station 106. The microprocessor controller 90 is further programmed for receiving, directly or through the memory, information from the pressure gauge 124 and from the four pairs of photocells 136, 138 and for controlling the centrifuge motor 80, the stepper motor 116 of the pumping station 106, and the four sets of pinch valves 126, 128, 130, 132 so as to cause the separation apparatus to operate along a selected separation protocol. The controller 90 also recognizes if one or more dummy bags have been placed in particular separation cells 78.

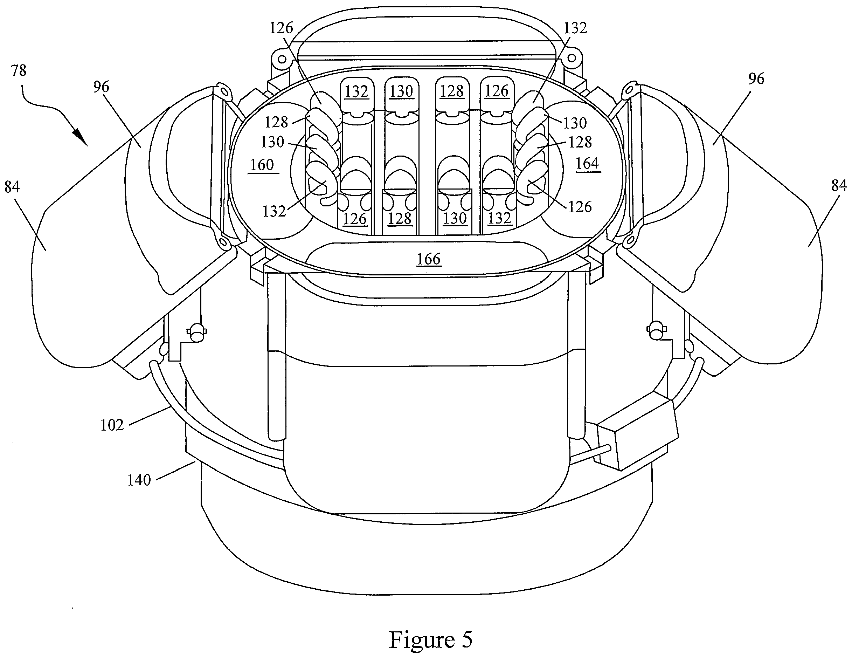

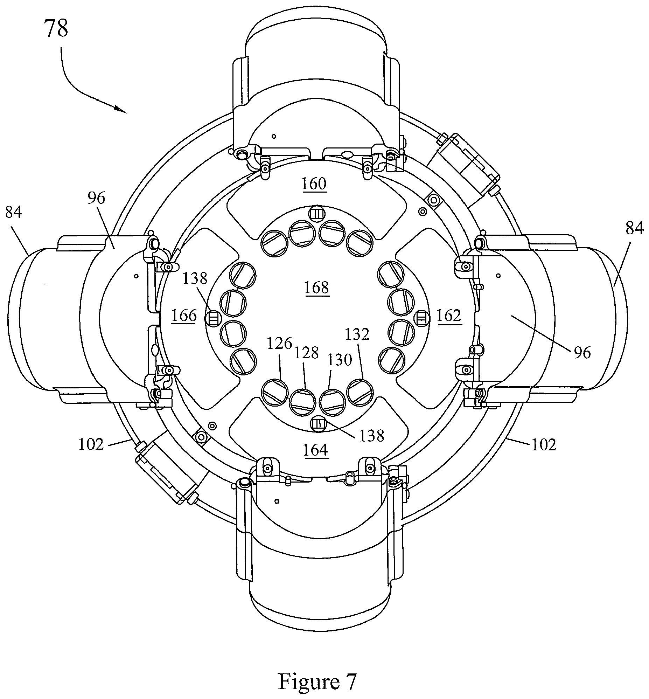

Figures 5, 6, and 7 show the rotor of a second embodiment of a separation apparatus for four discrete volumes of a composite liquid. The rotor of this second embodiment essentially differs from the rotor of the embodiment of Figures 2 and 3 in the spatial arrangement of the pinch valves 126, 128, 130, 134 and of the storage means for the component bags with respect to the separation cells 78. In this embodiment, the storage means, instead of comprising a central container, comprises four component containers 160, 162, 164, 166 that are arranged around a central cylindrical cavity 168, in which the four sets of pinch valves 126, 128, 130, 132 are mounted with their longitudinal axes parallel to the rotation axis 68. The cavity 88 of a component container 160, 162, 164, 166 has a regular bean-like cross-section. When a set of bag as shown in Figure 1 is mounted on the rotor of Figures 5, 6, and 7, the separation bag 12, the component bags 14, 16, the wash solution bag 44, and the wash solution discard bag 52 are located beyond the associated pinch valves 126, 128, 130, 132 with respect to the rotation axis 68. The tubes 32, 36, 46, 50 are then in the position shown in Figure 1.

The operation of the separation apparatus, in accordance with an illustrative separation protocol, will now be described. According to an illustrative protocol, four discrete volumes of blood are separated into a plasma component, a platelet component and a red blood cell component. Each volume of blood is contained in a separation bag 12 of a bag set represented in Figure 1, in which it has previously been collected from a donor using the collection tube 20. After the blood collection, the collection tube 20 has been sealed and cut close to the separation bag 12. Typically, the volumes of blood are not the same in the four separation bags 12, which, consequently, have slightly different weights. Also, typically, the hematocrit varies from one separation bag 12 to another.

### First stage: setting the four bag sets in the separation apparatus.

Separation bags 12 or dummy bags 170, as described below, are loaded into the four separation cells 78. The lids 96 are closed and locked, whereby the separation bags 12 are secured by their upper edge to the containers 84 (the pins 170 of the securing means pass through the holes 24 in the upper corner of the separation bags 12 and engage the recesses 172).

The tubes 32 connecting the separation bags 12 to the plasma component bags 14, through the X connectors 34 are inserted in the groove 134 of the first pinch valves 126. The tubes 50 connecting the separation bags 12 to the wash solution discard bags 52, through the T connectors 48 are inserted in the groove 134 of the second pinch valves 128. The tubes 46 connecting the separation bags 12 to the wash solution bags 44, if washing is used, through the T connectors 48 are inserted in the groove 134 of the third pinch valves 130. The tubes 36 connecting the separation bags 12 to the platelet component bags 16, through the X connectors 34 are inserted in the groove 134 of the fourth pinch valves 132. The four plasma component bags 14, the four platelet component bags 16, the four wash solution bags 44, the four wash solution discard bags 52, the four red blood cell component bags 38 and the four leuko-reduction filters 40 are inserted in the central compartment 74 of the rotor or in the respective compartment 160, 162, 164, 166. The pinch valves 126, 128, 130, 132 are closed and the breakable stoppers 26 in the tubes 18 connecting the separation bags 12 to the X connectors 34 and the T connectors 48 are manually broken.

### Second stage: balancing the rotor in order to compensate for the difference in weights of the separation bags.

At the onset of the second stage, all the pinch valves 126, 128, 130, 132 are closed. The rotor is set in motion by the centrifuge motor 80 and its rotation speed increases steadily until it rotates at a first centrifugation speed (high sedimentation speed or "hard spin"). Weights within the balance ring 140 shift within the ring 140 to balance the rotor, whatever the specific weights of the separation bags 12 that are loaded in the separation cells 78 may be. This does not imply that the internal cavity 88 of the separation cells 78 should be filled up at the end of the balancing stage. It does not matter if an empty space remains in each separation cell 78. The size of this empty space essentially depends on the volume of the internal cavity 88 of a separation cell 78 and the average volume of a blood donation.

### Third stage: the blood within the separation bags is sedimented to a desired level.

At the onset of this stage, all pinch valves 126, 128, 130, 132 are closed. The valves 126, 128, 130, 132 are all opened and the rotor is rotated at a second centrifugation speed (low sedimentation speed or "soft spin") for a predetermined period of time. The inner plasma layer does not substantially contain any cells, and the platelets and the white blood cells form an intermediary layer between the red blood cell layer and the plasma layer.

### Fourth stage: a first, larger, portion of plasma is transferred into the plasma bags, while a second, smaller, portion of plasma remains in the separation bags.

At the onset of this stage, the rotation speed is decreased to a third centrifugation speed. All four pinch valves 126 controlling access to the plasma component bags 14 are opened and the pumping station 106 is actuated to pump hydraulic liquid into the hydraulic chambers 100. As fluid is detected at each of the line sensors 138, the pinch valve 126 associated with that sensor 138 is closed. This process is continued until all four pinch valves 126 have been closed. The pumping station 106 is stopped.

A first pinch valve of the four first pinch valves 126 controlling access to a first plasma component bag 14 is opened, and the pumping station 106 is actuated to pump hydraulic liquid at a first constant flow rate into the hydraulic chambers 100 and constantly squeeze a first separation bag 12, causing the transfer of plasma into the first plasma component bag 14. The pinch valve 126 is closed. The volume of the plasma expressed into the first component bag 14 is computed from the volume of hydraulic fluid needed to express the plasma out of the separation bag.

Next, a third pinch valve of the four first pinch valves 126, diametrically across from the first pinch valve and first separation bag, is opened, and the pumping station 106 is actuated to pump hydraulic liquid at the first constant flow rate into the hydraulic chambers 100 and constantly squeeze a third separation bag 12, causing the transfer of plasma into a third plasma component bag 14. The pinch valve 126 is closed. The volume of the plasma expressed into the third component bag 14 is again computed from the volume of the additional hydraulic fluid needed to express the plasma out of the third separation bag.

Thereafter, a second pinch valve of the four first pinch valves 126, orthogonal from the first and third pinch valves and first and third separation bags, is opened, and the pumping station 106 is actuated to pump hydraulic liquid at the first constant flow rate into the hydraulic chambers 100 and constantly squeeze a second separation bag 12, causing the transfer of plasma into a second plasma component bag 14. The pinch valve 126 is closed. The volume of the plasma expressed into the second component bag 14 is again computed from the volume of the additional hydraulic fluid needed to express the plasma out of the second separation bag.

Finally, a fourth pinch valve of the four first pinch valves 126, diametrically across from the second pinch valve and second separation bag, is opened, and the pumping station 106 is actuated to pump hydraulic liquid at the first constant flow rate into the hydraulic chambers 100 and constantly squeeze a fourth separation bag 12, causing the transfer of plasma into a fourth plasma component bag 14. The pinch valve is closed. The volume of the plasma expressed into the fourth component bag 14 is again computed from the volume of the additional hydraulic fluid needed to express the plasma out of the fourth separation bag.

When blood cells are first detected by the bag sensor 136 in any of the separation cells 78, the pumping station 106 stops pumping hydraulic liquid and the corresponding pinch valve 126 is closed, as explained above. The expression of plasma from each separation bag 12 into the attached plasma component bag 14 is stopped immediately after detection of blood cells by the corresponding bag sensor 136, so that the volume of plasma remaining in the separation bag 12 is large enough to allow the platelets to be re-suspended therein.

### Fifth stage: a platelet component is prepared in the separation bags.

At the onset of this fifth stage, all the valves 126, 128, 130, and 132 are closed. The valves 126, 128, 130 and 132 are all opened and the rotor is stopped. The pumping station 106 is actuated to pump a volume of hydraulic liquid from the hydraulic chambers 100 at a high flow rate. The rotor is then controlled so as to oscillate back and forth around the rotation axis 68 for a determined period of time, at the end of which the cells in the separation bags 12 are substantially suspended in plasma. The rotor is then set in motion again by the centrifuge motor 80 so that its rotation speed increases steadily until it reaches a fourth centrifugation speed (low sedimentation speed or "soft spin"). The rotor is rotated at the fourth rotation speed for a predetermined period of time that is selected so that the blood components in the separation bags 12 at the end of the selected period are separated to a point where the separation bags 12 exhibit an outer layer comprising packed red blood cells and an inner annular layer substantially comprising platelets suspended in plasma.

### Sixth stage: a platelet component is transferred into the platelet bags.

At the onset of this stage, the rotation speed remains the same (fourth centrifugation speed). A first valve of the four fourth pinch valves 132 controlling access to the platelet bags 16 is opened, and the pumping station 106 is actuated so as to pump hydraulic liquid at a third constant flow rate into the hydraulic chambers 100 and consequently squeeze the separation bag 12 in the separation cell 78 associated with the opened fourth pinch valve 132 and cause the transfer of the platelets into the platelet component bag 16 connected to this separation bag 12.

After a predetermined period of time after blood cells are detected by the tube sensor 138 in the separation cell 78 associated with the opened fourth pinch valve 132, the pumping station 106 is stopped and the fourth pinch valve 132 is closed. The volume of the expressed plasma can be calculated from the volume of hydraulic fluid necessary to express the plasma.

After the first of the fourth pinch valves 132 has closed (i.e. the first pinch valve of the group of fourth pinch valves 132), a third one of the set of fourth pinch valves 132 is opened, diametrically across from the first pinch valve of the group of pinch valves 132, and a second platelet component is transferred into a platelet component bag 16, in the same manner as above. The same process is successively carried out to transfer the platelet component from the two remaining separation bags 12 into the platelet component bag 16 connected thereto, beginning with a second pinch valve 132 and separation bag 12 orthogonal to the first and third valves 132 and bags 12, and ending with a fourth valve 132 and bag 12, diametrically across from the second valve 132 and bag 12.

In the platelet component transfer process described above, the transfers of the four platelet components are successive, and the order of succession is predetermined. However, each of the second, third and four transfers starts following the occurrence of a specific event at the end of the previous transfer (detection of blood cells by the tube sensor 138 or closing of the second valve 132). As a variant, when the fourth flow rate is sufficiently low and the closing of the fourth pinch valves 132 occurs almost simultaneously with the detection of blood cells in the tubes 18, then the pumping station can be actuated continuously during the sixth stage. The sixth stage ends when the all four of the fourth pinch valves 132 are closed.

### Optional Seventh stage: the red blood cells are washed to remove prions.

In the optional seventh, or washing, stage, the packed red blood cells remaining in the separation bag 12 are washed one or more times to remove prions -- a disease-causing agent that is neither bacterial nor fungal nor viral and contains no genetic material.

With the four valves 126, 128, 130, 132 of each set of valves closed, the rotor of the centrifuge slows to a pre-determined low speed. As directed by the controller 90, the pump station 106 withdraws hydraulic fluid from the hydraulic chambers 100. The wash solution (or third) valve 130 opens, allowing wash solution to flow into the separation bag 12 by action of the centrifugal gravitational field. When sufficient wash solution has drained into the separation bag 12, the wash solution valve 130 closes and the centrifuge is further slowed. The red blood cell valves 86 are opened. The pump station 106 withdraws additional hydraulic fluid from the hydraulic chambers 100, creating a free surface above the fluids in the separation chamber.

The rotor is then controlled so as to oscillate back and forth around the rotation axis 68 for a determined period of time, at the end of which the cells in the separation bags 12 are substantially suspended in wash solution with a small amount of residual plasma. The rotor is then set in motion again by the centrifuge motor 80 so that its rotation speed increases steadily until it reaches the fourth centrifugation speed (low sedimentation speed or "soft spin"). The rotor is rotated at the fourth rotation speed for a predetermined period of time that is selected so that the blood components in the separation bags 12 at the end of the selected period are separated to a point where the separation bags 12 exhibit an outer layer comprising packed red blood cells and an inner annular layer substantially comprising wash solution with dilute plasma.

A first valve of the four discard (or second) pinch valves 128 controlling access to the wash solution discard bags 52 is opened, and the pumping station 106 is actuated so as to pump hydraulic liquid at a constant flow rate into the hydraulic chambers 100 and consequently squeeze the separation bag 12 in the separation cell 78 associated with the opened discard pinch valve 128 and cause the transfer of the used wash solution into the wash solution discard bag 52 connected to this separation bag 12. When blood cells are detected by the tube sensor 138 near the separation cell 78 associated with the opened discard pinch valve 128, the pumping station 106 is stopped and the discard pinch valve 128 is closed. After the first of the discard pinch valves 128 has closed (i.e. the first pinch valve of the group of discard pinch valves 128), a third one of the set of fourth pinch valves 128 is opened, diametrically across from the first of the discard pinch valves, and a third used wash solution is transferred into a wash solution discard bag 52, in the same manner as above.

The same process is successively carried out to transfer the used wash solution from the two remaining separation bags 12 into the wash solution discard bag 52 connected thereto, beginning with a second discard pinch valve 128 orthogonal to the first and third discard pinch valves and ending with a fourth discard pinch valve diametrically across from the second discard pinch valve. This seventh or washing stage may be repeated until all available wash solution has been used.

### Eighth stage: the centrifugation process is ended.

The controller 90 is programmed to start the eighth stage after all four of the discard pinch valves 128 are closed, upon receiving information from the last tube sensor 138 to detect blood cells. The rotation speed of the rotor is decreased until the rotor stops, the pumping station 106 is actuated so as to pump the hydraulic liquid from the hydraulic chambers 100 at a high flow rate until the hydraulic chambers 100 are empty, and the first and fourth pinch valves 126, 132 are actuated so as to seal and cut the tubes 32, 36. The second and third pinch valves 128, 130 may also be actuated so as to seal and cut the tubes 46, 50, thereby isolating the residual wash solution and the discarded wash solution. The red blood cells remain in the separation bags 12. When the eighth stage is completed, the four bag sets are removed from the separation apparatus and each bag set is separately handled manually.

The breakable stopper 28 blocking the communication between the separation bag 12 and the tube 22 connected thereto is broken, as well as the red cell collection bag 38 and the tube 22. The storage solution contained in the red cell collection bag 38 is allowed to flow by gravity through the leuko-reduction filter 40 and into the separation bag 12, where it is mixed with the red blood cells so as to lower the viscosity thereof. The content of the separation bag 12 is then allowed to flow by gravity through the filter 40 and into the red cell component bag 38. The any residual white blood cells are trapped by the filter 40, so that substantially only red blood cells are collected into the red cell component bag 38.

### Use of a Dummy Bag

According to the present invention, a dummy bag 170, containing an inert fluid such as water or saline solution, is placed in one or more of the processing locations to replace a separation or whole blood bag 12. The dummy bag has characteristics that allow the blood processing apparatus to recognize the presence of the dummy bag. The blood processing apparatus then performs separation and optional washing procedures only at those processing areas containing blood components, and not on the areas containing a dummy bag.

Like the separation bag 12, the dummy bag 170 is flat and generally rectangular. It may be made of two sheets of plastic material that are welded together so as to define there between an interior space having a main rectangular portion connected to a triangular top portion. The dummy bag may also be formed of a unitary block of a suitable substance such as silicone, urethane, or polyurethane. A first tube 172 is connected to the top of the triangular portion of the dummy bag 170. The dummy bag 170 further comprises a hole 174 in each of its two proximal corners that are adjacent to the three tubes 18, 20, 22. The holes 24 are used to secure the separation bag to a separation cell, as will be described later.

The dummy bag may contain a volume of inert liquid, such as water or saline solution, or a material such as silicon, having a density similar to whole blood, such that the weight of the dummy bag is within limits for units of whole blood that can be processed at a separation cell. The material in the dummy bag may comprise polymer granules which may be suspended in liquid.

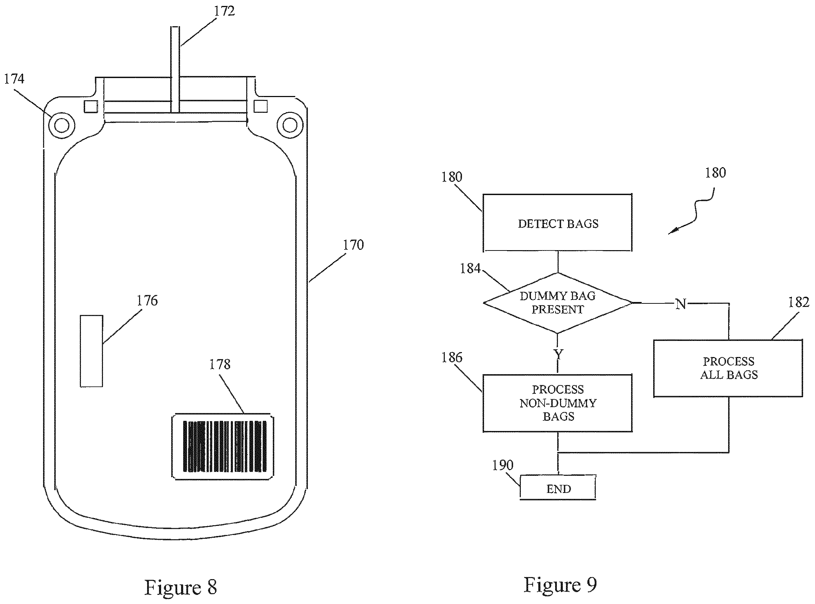

Several methods for dummy bag recognition may be used. In the apparatus described herein, photo sensors 136a, 136b, or 138 are used to control the separation process at each separation area. In one embodiment, an opaque dummy bag may be detected by a photo sensor 136a or 136b located in the container 84 for cell separation. In another embodiment, the short stub of tubing 172 is adapted to be placed next to the tubing photo sensor 138, which is otherwise used to detect the presence of various blood components, as described above. The stub of tubing 172 may be opaque or have optical characteristics that are unique as compared to blood or a blood component. In yet another embodiment, a specialized sensor, such as a radio frequency sensor (not shown), on the blood processing apparatus, may be used to detect a passive radio frequency emitter or transponder 176 on the dummy bag. In a further embodiment, the dummy bag may have a bar code label 178 that could be read by a bar code reader used to track units of blood through a separation process, as is known in the art. Signals from the sensors 136a, 136b, or 138, or from the radio frequency transponder 176, or from reading the bar code label 178 are transmitter to the controller 90. Once the controller 90 of the blood processing apparatus has determined that a dummy bag has been placed in one or more blood processing areas, the apparatus will not attempt to process the areas with a dummy bag, as shown in the schematic diagram 180 of Fig. 9. Hydraulic fluid may be displaced into the separation cell containing the dummy bag for balancing purposes, but the apparatus will not attempt to process blood at the identified separation cell. The controller 90 will test 182 each container 84 for the presence of a bag. If a dummy bag is detected 184, only the apparatus in those containers containing a whole blood or separation bag 12 will be activated to process the whole blood in the bags in those separation cells, as described above. Otherwise, all of the available separation bags will be processed 188 to completion 190.

It will be apparent to those skilled in the art that various modifications can be made to the apparatus and method described herein. Thus, it should be understood that the invention is not limited to the subject matter discussed in the specification. Rather, the present invention is intended to cover modifications and variations falling within the scope of the claims.

## Claims

1. A method for separating at least one volume of blood into at least a first component and a second component, said method comprising:
enclosing in each of at least two separation cells (78) mounted on a rotor (64) either a separation bag (12) containing blood or a dummy bag (170) having a weight approximating a separation bag (12) containing blood, said dummy bag (170) containing no blood or blood components;
storing in at least one container (160, 162, 164, 166) included in the rotor (64) at least one first component bag (14, 16) connected to the at least one separation bag(12);
determining if any bag in any separation cell (78) is a dummy bag;(170);
rotating the rotor (64) at a sedimentation speed at which the at least first and second components sediment in each of the separation bags (12) containing blood;
transferring at least one fraction of a first separated component from each of the separation bags (12) in separation cells (78) not containing a dummy bag (170) into the at least one first component bags (14, 16) connected thereto respectively.

2. The method of claim 1, wherein the step of determining if any bag is a dummy bag further comprises detecting an opaque bag using a photo sensor in the separation cell.

3. The method of claim 1, wherein the step of determining if any bag is a dummy bag further comprises detecting an opaque tube segment attached to a dummy bag using a photo sensor on the rotor.

4. The method of claim 1, wherein the step of determining if any bag is a dummy bag further comprises detecting a radio frequency transponder on a dummy bag.

5. The method of claim 1, wherein the step of determining if any bag is a dummy bag further comprises detecting a bar code on a dummy bag.

6. A blood processing apparatus (60) for separating at least two discrete volumes of a composite liquid into at least a first component and a second component, the apparatus comprising:
a centrifuge (62) comprising a rotor (64) with a rotation axis (68);
at least two separation cells (78) mounted on the rotor (64);
at least one sensor (136a, 136b) associated with each separation cell (78) for generating information related to a characteristic of a component separated in a separation bag within the separation cell (78); and
a control unit (90) programmed for receiving information generated by the at least one sensor (136a, 136b) associated with each separation cell (78) and for controlling rotation speed in view of information generated by the at least one sensor (136a, 136b), **characterized in that**:
the at least one sensor (136a, 136b) can detect a dummy bag (170) in the separation cell (78); and
when the control unit (90) has determined that a dummy bag (170) has been placed in a separation cell (78) the apparatus will not attempt to process the separation cell (78) with a dummy bag (170).

7. The apparatus of claim 6, wherein the at least one sensor is a photo sensor that detects the dummy bag.

8. The apparatus of claim 6, wherein the control unit does not process blood at an identified separation cell that contains the dummy bag.

9. The apparatus of claim 6, further comprising a radio frequency sensor for detecting a radio frequency emitter on the dummy bag.

10. The apparatus of claim 6, further comprising a dummy bag (170) having a weight approximating a separation bag (12) containing blood, said dummy bag (170) containing no blood or blood components and comprising means for allowing said blood processing apparatus (60) to distinguish said dummy bag (170) from a blood-containing separation bag (160, 162, 164, 166).

11. The apparatus of claim 10, wherein at least part of said dummy bag is opaque.

12. The apparatus of claim 10, wherein said dummy bag further comprises stub tubing attached to said bag and said stub tubing is opaque.

13. The apparatus of claim 10, wherein said dummy bag comprises a bar code label.

14. The apparatus of claim 10, wherein said dummy bag comprises a radio frequency transponder.

## Patentansprüche

1. Verfahren zum Trennen von mindestens einem Blutvolumen in mindestens eine erste Komponente und eine zweite Komponente, wobei das Verfahren Folgendes umfasst:
Einschließen in jeder von mindestens zwei Trennzellen (78), die an einem Rotor (64) montiert sind, von entweder einem Trennbeutel (12), der Blut enthält, oder einem Dummy-Beutel (170), der ein Gewicht aufweist, das ungefähr einem Trennbeutel (12), der Blut enthält, entspricht, wobei der Dummy-Beutel (170) kein Blut oder keine Blutkomponenten enthält;
Speichern in mindestens einem Behälter (150, 162, 164, 166), der in dem Rotor (64) beinhaltet ist, mindestens eines ersten Komponentenbeutels (14, 16), der mit dem mindestens einen Trennbeutel (12) verbunden ist;
Bestimmen, ob ein beliebiger Beutel in einer Trennzelle (78) ein Dummy-Beutel (170) ist;
Drehen des Rotors (64) bei einer Ablagerungsgeschwindigkeit, bei der sich die mindestens eine erste und zweite Komponente in jedem der Trennbeutel (12), die Blut enthalten, ablagern;
Übertragen mindestens einer Fraktion einer ersten getrennten Komponente von jedem der Trennbeutel (12) in Trennzellen (78), die keinen Dummy-Beutel (170) enthalten, in den mindestens einen ersten Komponentenbeutel (14, 16), der jeweils damit verbunden ist.

2. Verfahren nach Anspruch 1, wobei der Schritt des Bestimmens, ob ein beliebiger Beutel ein Dummy-Beutel ist, ferner ein Erfassen eines undurchsichtigen Beutels unter Verwendung eines Fotosensors in der Trennzelle umfasst.

3. Verfahren nach Anspruch 1, wobei der Schritt des Bestimmens, ob ein beliebiger Beutel ein Dummy-Beutel ist, ferner ein Erfassen eines undurchsichtigen Rohrsegments, das an einem Dummy-Beutel abgebracht ist, unter Verwendung eines Fotosensors an dem Rotor umfasst.

4. Verfahren nach Anspruch 1, wobei der Schritt des Bestimmens, ob ein beliebiger Beutel ein Dummy-Beutel ist, ferner ein Erfassen eines Hochfrequenztransponders an einem Dummy-Beutel umfasst.

5. Verfahren nach Anspruch 1, wobei der Schritt des Bestimmens, ob ein beliebiger Beutel ein Dummy-Beutel ist, ferner ein Erfassen eines Barcodes an einem Dummy-Beutel umfasst.

6. Blutverarbeitungsvorrichtung (60) zum Trennen von mindestens zwei unterschiedlichen Volumen einer zusammengesetzten Flüssigkeit in mindestens eine erste Komponente und eine zweite Komponente, wobei die Vorrichtung Folgendes umfasst:
eine Zentrifuge (62), umfassend einen Rotor (64) mit einer Drehachse (68);
mindestens zwei Trennzellen (78), die an dem Rotor (64) montiert sind;
mindestens einen Sensor (136a, 136b), der jeder Trennzelle (78) zugeordnet ist, zum Erzeugen von Informationen in Bezug auf ein Merkmal einer Komponente, die in einem Trennbeutel in der Trennzelle (78) getrennt ist; und
eine Steuereinheit (90), die zum Empfangen von Informationen programmiert ist, die durch den mindestens einen Sensor (136a, 136b) erzeugt werden, der jeder Trennzelle (78) zugeordnet ist, und zum Steuern einer Drehgeschwindigkeit im Hinblick auf Informationen, die durch den mindestens einen Sensor (136a, 136b) erzeugt werden, **dadurch gekennzeichnet, dass**:
der mindestens eine Sensor (136a, 136b) einen Dummy-Beutel (170) in der Trennzelle (78) erfassen kann; und
wenn die Steuereinheit (90) bestimmt hat, dass ein Dummy-Beutel (170) in einer Trennzelle (78) platziert wurde, die Vorrichtung nicht versuchen wird, die Trennzelle (78) mit einem Dummy-Beutel (170) zu verarbeiten.

7. Vorrichtung nach Anspruch 6, wobei der mindestens eine Sensor ein Fotosensor ist, der den Dummy-Beutel erfasst.

8. Vorrichtung nach Anspruch 6, wobei die Steuereinheit Blut nicht an einer identifizierten Trennzelle, die den Dummy-Beutel enthält, verarbeitet.

9. Vorrichtung nach Anspruch 6, ferner umfassend einen Hochfrequenztransponder zum Erfassen eines Hochfrequenzsenders an dem Dummy-Beutel.

10. Vorrichtung nach Anspruch 6, ferner umfassend einen Dummy-Beutel (170) mit einem Gewicht, das ungefähr einem Trennbeutel (12), der Blut enthält, entspricht, wobei der Dummy-Beutel (170) kein Blut oder keine Blutkomponenten enthält und Mittel zum Ermöglichen der Blutverarbeitungsvorrichtung (60), den Dummy-Beutel (170) von einem Blut enthaltenden Trennbeutel (160, 162, 164, 166) zu unterscheiden, umfasst.

11. Vorrichtung nach Anspruch 10, wobei mindestens ein Teil des Dummy-Beutels undurchsichtig ist.

12. Vorrichtung nach Anspruch 10, wobei der Dummy-Beutel ferner einen Rohrstutzen umfasst, der an dem Beutel angebracht ist, und der Rohrstutzen undurchsichtig ist.

13. Vorrichtung nach Anspruch 10, wobei der Dummy-Beutel ein Barcode-Etikett umfasst.

14. Vorrichtung nach Anspruch 10, wobei der Dummy-Beutel einen Hochfrequenztransponder umfasst.

## Revendications

1. Procédé de séparation d'au moins un volume de sang en au moins un premier composant et un deuxième composant, ledit procédé comprenant :
l'inclusion dans chacune d'au moins deux cellules de séparation (78) montées sur un rotor (64) soit une poche de séparation (12) contenant du sang ou une poche vide (170) présentant un poids proche d'une poche de séparation (12) contenant du sang, ladite poche vide (170) ne contenant pas de sang ou de composant sanguin ;
le stockage dans au moins un récipient (160, 162, 164, 166) compris dans le rotor (64) d'au moins une première poche de composants (14, 16) reliée à l'au moins une poche de séparation (12) ;
la détermination de savoir si une quelconque poche dans une quelconque cellule de séparation (78) est une poche vide (170) ;
la rotation du rotor (64) à une vitesse de sédimentation à laquelle les au moins premier et deuxième composants se sédimentent dans chacune des poches de séparation (12) contenant du sang ;
le transfert d'au moins une fraction d'un premier composant séparé depuis chacune des poches de séparation (12) dans des cellules de séparation (78) ne contenant pas une poche vide (170) vers les poches (14, 16) de l'au moins un premier composant reliées à celles-ci respectivement.

2. Procédé selon la revendication 1, dans lequel l'étape de détermination de savoir si une quelconque poche est une poche vide comprend en outre la détection d'une poche opaque à l'aide d'un photocapteur dans la cellule de séparation.

3. Procédé selon la revendication 1, dans lequel l'étape de détermination de savoir si une quelconque poche est une poche vide comprend en outre la détection d'un segment de tube opaque attaché à une poche vide à l'aide d'un photocapteur sur le rotor.

4. Procédé selon la revendication 1, dans lequel l'étape de détermination de savoir si une quelconque poche est une poche vide comprend en outre la détection d'un transpondeur radiofréquence sur une poche vide.

5. Procédé selon la revendication 1, dans lequel l'étape de détermination de savoir si une quelconque poche est une poche vide comprend en outre la détection d'un code barres sur une poche vide.

6. Appareil de traitement de sang (60) destiné à séparer au moins deux volumes discrets d'un liquide composite en au moins un premier composant et un deuxième composant, l'appareil comprenant :
une centrifugeuse (62) comprenant un rotor (64) avec un axe de rotation (68) ;
au moins deux cellules de séparation (78) montées sur le rotor (64) ;
au moins un capteur (136a, 136b) associé à chaque cellule de séparation (78) en vue de générer des informations liées à une caractéristique d'un composant séparé dans une poche de séparation à l'intérieur de la cellule de séparation (78) ; et
une unité de commande (90) programmée pour recevoir des informations générées par l'au moins un capteur (136a, 136b) associé à chaque cellule de séparation (78) et permettant de commander la vitesse de rotation au vu des informations générées par l'au moins un capteur (136a, 136b), **caractérisé en ce que** :
l'au moins un capteur (136a, 136b) peut détecter une poche vide (170) dans la cellule de séparation (78) ; et
lorsque l'unité de commande (90) a déterminé qu'une poche vide (170) a été placée dans une cellule de séparation (78) l'appareil ne tente pas de traiter la cellule de séparation (78) avec une poche vide (170).

7. Appareil selon la revendication 6, dans lequel l'au moins un capteur est un photocapteur qui détecte la poche vide.

8. Appareil selon la revendication 6, dans lequel l'unité de commande ne traite pas le sang au niveau d'une cellule de séparation identifiée qui contient la poche vide.

9. Appareil selon la revendication 6, comprenant en outre un capteur de radio fréquences destiné à détecter un émetteur de radio fréquences sur la poche vide.

10. Appareil selon la revendication 6, comprenant en outre une poche vide (170) présentant un poids proche d'une poche de séparation (12) contenant du sang, ladite poche vide (170) ne contenant pas de sang ou de composant sanguin et comprenant un moyen permettant audit appareil de traitement de sang (60) de distinguer ladite poche vide (170) d'une poche de séparation contenant du sang (160, 162, 164, 166).

11. Appareil selon la revendication 10, dans lequel au moins une partie de ladite poche vide est opaque.

12. Appareil selon la revendication 10, dans lequel ladite poche vide comprend en outre une tubulure à embouts attachée à ladite poche et ladite tubulure à embouts étant opaque.

13. Appareil selon la revendication 10, dans lequel ladite poche vide comprend une étiquette de code barres.

14. Appareil selon la revendication 10, dans lequel ladite poche vide comprend un transpondeur radiofréquence.
